# EUROPEAN PATENT APPLICATION

(11) **EP 4 331 371 A1**
(43) Date of publication of application: **06.03.2024**
(21) Application number: 22795593.7
(22) Date of filing: 14.04.2022
(51) Int. Cl.: A23D 9/00, A23L 27/00

(54) **FLAVOR-IMPARTING AGENT AND METHOD FOR PRODUCING SAME**

(30) Priority: 28.04.2021 JP 2021076196
(71) Applicant: THE NISSHIN OILLIO GROUP, LTD., Tokyo 104-8285 (JP)
(72) Inventor: AOYAGI, Kanji, Yokohama-shi, Kanagawa 235-8558 (JP); SEKIGUCHI, Yoshinori, Yokohama-shi, Kanagawa 235-8558 (JP)
(74) Representative: Hasegawa, Kan
(86) International application number: PCT/JP2022/017821
(87) International publication number: WO 2022/230679

(57) **Abstract**

The present invention provides a flavor-imparting agent, based on oils and/or fats, with an improved flavor, particularly a sweet scent and/or milky scent presented by lactones having 6 to 14 carbon atoms.

The present invention makes it possible to obtain a flavor-imparting agent with a sweet scent and/or milky scent by producing a specified quantity of lactones having 6 to 14 carbon atoms through oxidizing and heating glycerides, having specific constituent fatty acids, at 140°C or above.

## Description

### Technical Field

The present invention relates to a flavor-imparting agent that includes specific glycerides and lactones, as well as a method for producing the same.

### Background Art

Oils and/or fats (lipids) are not only one of the three major nutrients, but also crucial ingredients that impart deliciousness to foods. Oils and/or fats make the flavor of food mild and provide richness. Foods that become tasty because of oils and/or fats include, for example, tempura, fried foods, ramen, pies, chocolate, creams, and cheese. Oils and/or fats include various oils and/or fats ranging from nearly tasteless and odorless oils and/or fats such as salad oil to oils and/or fats such as roasted sesame oil and butter, which are valued for their unique flavors. These oils and/or fats are used according to their application.

As a method for improving the flavor of oils and/or fats compositions, particularly butter, a method is known for enriching lactones in the oils and/or fats composition non-enzymatically (Patent literature 1). Specifically, Patent Literature 1 discloses a method for producing a lactone-enriched oils and/or fats composition, which includes maintaining an oils and/or fats composition with a moisture content of 300 ppm or more and 1200 ppm or less at a temperature where solid fats exist in the oils and/or fats composition (preferably 10°C or more and 35°C or less), or adding an additive that has the property of forming hydrogen bonds with water molecules in the oils and/or fats composition, and maintaining it at a temperature of 40 to 60°C, to produce γ-lactone or δ-lactone non-enzymatically from triglycerides containing 4- or 5-hydroxy fatty acids in the oils and/or fats composition.

Furthermore, as a method for enhancing the flavor of oils and/or fats, particularly the base richness, a method is known for including a specific quantity of specific coconut oil in the oils and/or fats (Patent Literature 2). Specifically, Patent Literature 2 discloses oils and/or fats that contain 0.001 to 15% by mass of virgin coconut oil.

### Citation List

### Patent Literatures

Patent Literature 1: Republished Japanese Patent No. 2013/105624
Patent Literature 2: Japanese Patent Application Publication No. 2017-213004

### Summary of Invention

### Problems to be solved by the invention

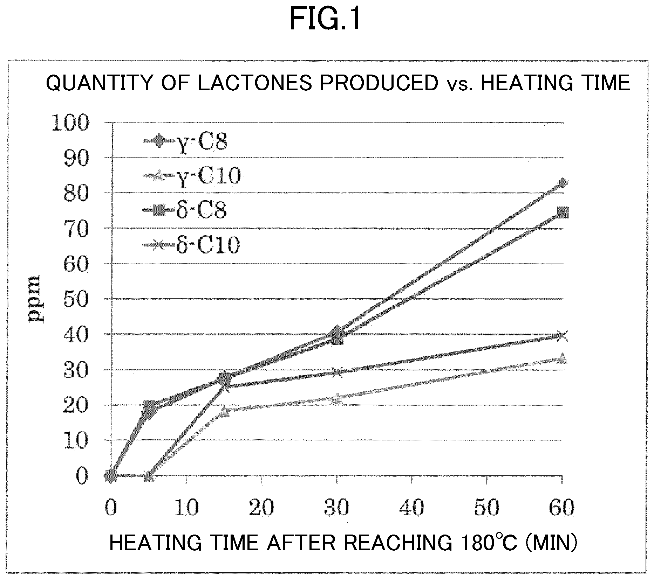

However, the production method in Patent Literature 1 is a production method that requires the presence of 4- or 5-hydroxy fatty acids as shown in Claim 1 and Figure 1 of Patent Literature 1, and under conditions where these fatty acids are not present, lactones are not sufficiently produced. Moreover, the virgin coconut oil contained in the oils and/or fats composition of Patent Literature 2 contains a large quantity of unsaturated fatty acids, which are the cause of rancid odor, and therefore the scent of lactones in virgin coconut oil is not sufficiently perceived.

An object of the present invention is to provide a flavor-imparting agent that is based on oils and/or fats, has improved flavor, and especially has the sweet scent and/or the milky scent that is given off by lactones having 6 to 14 carbon atoms.

### Means for solution of the problems

The present inventors have intensively studied to achieve the above object and found that by oxidatively heating a glyceride having specific constituent fatty acids at 140°C or higher to produce a specific quantity of lactones having 6 to 14 carbon atoms, it is possible to obtain a flavor-imparting agent that has a sweet scent and/or milky scent. This led to the completion of the present invention.

Specifically, the present invention includes the following aspects.
[1] A method for producing a flavor-imparting agent, comprising:
   oxidation heat treatment of glycerides at 140°C or higher to produce lactones having 6 to 14 carbon atoms, thereby preparing a flavor-imparting agent containing 5 mass ppm or more of the lactones having 6 to 14 carbon atoms, wherein
   10% by mass or more of constituent fatty acids of the glycerides are straight-chain saturated fatty acids having 6 to 14 carbon atoms, and
   the lactones are γ-lactones and/or δ-lactones.
[2] The method according to [1], wherein 0 to 5% by mass of the constituent fatty acids of the glycerides are unsaturated fatty acids.
[3] The method according to [1] or [2], wherein 60% by mass or more of the constituent fatty acids of the glycerides are straight-chain saturated fatty acids having 6 to 14 carbon atoms.
[4] The method according to any one of [1] to [3], wherein the constituent fatty acids of the glycerides are composed only of straight-chain saturated fatty acids having 6 to 14 carbon atoms.
[5] The method according to any one of [1] to [4], wherein the glycerides are triglycerides.
[6] The method according to any one of [1] to [5], further comprising: mixing additional glycerides after the oxidation heat treatment, to thereby prepare a flavor-imparting agent containing 5 ppm or more of lactones having 6 to 14 carbon atoms.
[7] A flavor-imparting agent comprising:
   80% by mass or more of glycerides; and
   5 ppm or more of lactones having 6 to 14 carbon atoms, based on a total mass of the flavor-imparting agent, wherein
   0 to 5% by mass of constituent fatty acids of the glycerides are unsaturated fatty acids,
   10% by mass or more of the constituent fatty acids of the glycerides are straight-chain saturated fatty acids having 6 to 14 carbon atoms, and
   the lactones are γ-lactones and/or δ-lactones.
[8] The flavor-imparting agent according to [7], wherein the glycerides have a peroxide value of 7.5 or higher and/or an anisidine value of 1.0 or higher.
[9] The flavor-imparting agent according to [7] or [8], wherein 60% by mass or more of the constituent fatty acids of the glycerides are straight-chain saturated fatty acids having 6 to 14 carbon atoms.
[10] The flavor-imparting agent according to any one of [7] to [9], wherein the constituent fatty acids of the glycerides are composed only of straight-chain saturated fatty acids having 6 to 14 carbon atoms.
[11] The flavor-imparting agent according to any one of [7] to [10], wherein the glycerides are triglycerides.
[12] The flavor-imparting agent according to any one of [7] to [11], which has a sweet scent and/or milky scent.
[13] A method for imparting flavor to glycerides, comprising:
   oxidation heat treatment of glycerides at 140°C or higher to produce 5 mass ppm or more of lactones having 6 to 14 carbon atoms, wherein
   10% by mass or more of constituent fatty acids of the glycerides are straight-chain saturated fatty acids having 6 to 14 carbon atoms, and
   the lactones are γ-lactones and/or δ-lactones.

### Advantageous Effects of Invention

According to the present invention, it is possible to provide a flavor-imparting agent that is based on oils and/or fats, has improved flavor, and especially has the sweet scent and/or the milky scent that is given off by lactones having 6 to 14 carbon atoms.

### Brief Description of Drawings

Fig. 1 illustrates the analysis results of the quantity of lactones produced and the time from when the sample temperature reached 180°C after heating the raw material glyceride (O.D.O) at 180°C.

### Description of Embodiments

The present invention relates to a method for producing a flavor-imparting agent, including the step of oxidation heat treatment of glycerides at 140°C or higher to produce lactones having 6 to 14 carbon atoms, thereby preparing a flavor-imparting agent containing 5 mass ppm or more of the lactones having 6 to 14 carbon atoms, wherein 10% by mass or more of constituent fatty acids of the glycerides are straight-chain saturated fatty acids having 6 to 14 carbon atoms, and the lactones are γ-lactones and/or δ-lactones.

In the glycerides serving as raw materials, 10% by mass or more of their constituent fatty acids, for example, 30% by mass or more, 60% by mass or more, 75% by mass or more, 90% by mass or more, or all (100% by mass) are straight-chain saturated fatty acids having 6 to 14 carbon atoms. The straight-chain saturated fatty acids having 6 to 14 carbon atoms may be of one type or of two or more types. The straight-chain saturated fatty acids having 6 to 14 carbon atoms include caproic acid, caprylic acid, capric acid, lauric acid, and myristic acid, preferably caprylic acid and/or capric acid, or lauric acid and/or myristic acid. More preferably, caprylic acid and/or capric acid is mentioned, and most preferably, caprylic acid is mentioned. If 10% by mass or more of the constituent fatty acids are straight-chain saturated fatty acids having 6 to 14 carbon atoms, a desired quantity of lactones having 6 to 14 carbon atoms can be produced. Note that the quantity of lactones produced is very small compared to the quantity of the constituent fatty acids of glycerides, so that the quantity of glycerides and the ratio of the constituent fatty acids (mass ratio) are hardly affected by the generation from fatty acids to lactones. Therefore, before and after the oxidation heat treatment, the quantity of glycerides and the mass ratio of the constituent fatty acids are substantially the same.

In cases where unsaturated fatty acids are included as constituent fatty acids in the glycerides, other than straight-chain saturated fatty acids having 6 to 14 carbon atoms, there is a risk that a rancid odor produced by the oxidation of the unsaturated fatty acids may be added to the flavor-imparting agent, so that it is preferable to have a smaller quantity of unsaturated fatty acids. For example, it is preferable that 0 to 5% by mass of the constituent fatty acids of the glycerides are unsaturated fatty acids, and it is more preferable that 0 to 3% by mass thereof are unsaturated fatty acids. In the case of including unsaturated fatty acids, it is preferable to have straight-chain unsaturated fatty acids having 16 to 22 carbon atoms, which constitute vegetable oils, and more preferably, one or more selected from oleic acid, linoleic acid, linolenic acid, and erucic acid.

The raw material glycerides may include one or more of monoglycerides, diglycerides, or triglycerides, and preferably, 50% by mass or more, 60% by mass or more, 70% by mass or more, 80% by mass or more, 90% by mass or more, 95% by mass or more, or 100% by mass of the raw material glycerides is triglyceride. These raw material glycerides can be obtained by an esterification reaction of fatty acids and glycerin, an interesterification reaction of fatty acid esters and glycerin, fatty acids and oils and/or fats, or the like, or an interesterification reaction of methyl or ethyl esters of fatty acids and glycerin or oils and/or fats. Also, when unsaturated fatty acids are included, unsaturated bonds may be reduced by hydrogenating fatty acids, methyl or ethyl esters of fatty acids, or glycerides. In addition, hydrogenated coconut oil or palm kernel oil can also be used. Note that it is possible to mix other oils and/or fats, for example, coconut oil, palm kernel oil, palm oil, palm fractionated oil (such as palm olein, palm super olein, palm mid fraction, and palm stearin), shea butter, shea fractionated oil, monkey butter, monkey fractionated oil, illipe butter, soybean oil, rapeseed oil, cottonseed oil, safflower oil, sunflower oil, rice oil, corn oil, sesame oil, olive oil, perilla oil, linseed oil, peanut oil, milk fat, cocoa butter, and other animal and vegetable oils and/or fats, or mixed oils thereof, as well as processed oils and/or fats and the like, within the range where 10% by mass or more of the constituent fatty acids of the raw material glycerides are straight-chain saturated fatty acids having 6 to 14 carbon atoms.

The raw glycerides used can preferably be those that have been refined for edible use, and a mixture of refined glycerides and unrefined glycerides can also be used. From the viewpoint of the flavor of the resulting flavor-imparting agent, the content of unrefined glycerides is preferably 0 to 20% by mass, more preferably 0 to 10% by mass, and further preferably 0 to 5% by mass, based on the total mass of the raw glycerides. Examples of the edible refining process include degumming, neutralizing, bleaching, and deodorizing.

The temperature in the oxidation heat treatment of the raw glycerides can be any temperature at which lactones having 6 to 14 carbon atoms are produced, and it is 140°C or higher, preferably 150°C to 250°C, more preferably 160°C to 225°C, and further preferably 170°C to 200°C. The heating time depends on the heating temperature, but it may be long enough to oxidize the raw glycerides and produce lactones. For example, if the heating temperature is 180°C or higher, after reaching 180°C, the raw glycerides may be heated for 5 minutes or more, preferably for 10 to 120 minutes, 20 to 90 minutes, or 45 to 75 minutes. Also, for example, if the heating temperature is 160°C to 180°C, after reaching 160°C, the raw glycerides may be heated for 10 minutes or more, preferably for 15 to 160 minutes, or 30 to 120 minutes. Moreover, for example, if the heating temperature is 140°C to 160°C, after reaching 140°C, the raw glycerides may be heated for 15 minutes or more, preferably for 90 to 180 minutes, or 90 to 160 minutes. There are no particular restrictions on the heating treatment conditions as long as they allow for the oxidation of the raw glycerides to produce lactones, and it is preferable to heat the raw glycerides under the presence of oxygen, for example, in the atmosphere.

Lactones with a carbon number corresponding to the constituent fatty acids in the raw material glyceride are produced by oxidation heat treatment. In the method of the present invention, lactones having 6 to 14 carbon atoms, preferably 8 to 14 carbon atoms, more preferably 8 to 10 carbon atoms, and further preferably 8 carbon atoms, are produced.

In the method of the present invention, the content of lactones having 6 to 14 carbon atoms produced in the flavor-imparting agent is 5 ppm or more, preferably 15 ppm or more, 30 ppm or more, 60 ppm or more, 90 ppm or more, 120 ppm or more, 150 ppm or more, 200 ppm or more, 250 ppm or more, or 300 ppm or more. The content of lactones having 6 to 14 carbon atoms in the flavor-imparting agent may be, for example, 600 ppm or less, 500 ppm or less, 400 ppm or less, or 350 ppm or less. If the content of lactones having 6 to 14 carbon atoms is 5 ppm or more, the flavor-imparting agent exhibits a sweet scent and/or milky scent. As long as the produced lactones having 6 to 14 carbon atoms have a quantity in this range, it is acceptable if lactones having 5 or less or 15 or more carbon atoms are produced.

As for lactones having 6 to 14 carbon atoms, stably structured γ-lactones and/or δ-lactones are produced. There are no particular restrictions on the mass ratio (γ:δ) of γ-lactones and δ-lactones having 6 to 14 carbon atoms, and for example, it may be 100:0 to 0:100. For example, in the case of the mass ratio (γ:δ) of γ-lactones and δ-lactones having 6 to 10 carbon atoms, it may be 90:10 to 10:90, 80:20 to 20:80, 70:30 to 30:70, 60:40 to 40:60, or 55:45 to 45:55. For example, in the case of the mass ratio (γ:δ) of γ-lactones and δ-lactones having 11 to 14 carbon atoms, it may be 50:50 to 0:100, 40:60 to 0:100, 30:70 to 0:100, 20:80 to 0:100, 10:90 to 0:100, or 5:95 to 0:100.

The method of the present invention may include a step of further mixing additional glycerides after the oxidation heat treatment of the raw material glycerides, to thereby prepare a flavor-imparting agent containing 5 ppm or more of lactones having 6 to 14 carbon atoms. The mixing of additional glycerides can be carried out using any conventionally known means without particular restriction. The additional glycerides may be the same as or different from the raw material glycerides, and may be any glycerides that can be typically added to food. The quantity of the additional glycerides mixed is not particularly limited, as long as the resulting flavor-imparting agent contains 5 ppm or more of the lactones having 6 to 14 carbon atoms, which is produced by the oxidation heat treatment.

The present invention also relates to a method for imparting flavor to glycerides, including the step of oxidation heat treatment of glycerides at 140°C or higher to produce 5 mass ppm or more of lactones having 6 to 14 carbon atoms, wherein 10% by mass or more of constituent fatty acids of the glycerides are straight-chain saturated fatty acids having 6 to 14 carbon atoms, and the lactones are γ-lactones and/or δ-lactones. The raw material glycerides, the produced lactones having 6 to 14 carbon atoms, and the conditions for the oxidation heat treatment are similar to those described above for the method for producing a flavor-imparting agent.

The present invention also relates to a flavor-imparting agent containing 80% by mass or more of glycerides and 5 ppm or more of lactones having 6 to 14 carbon atoms, based on a total mass of the flavor-imparting agent, wherein 0 to 5% by mass of constituent fatty acids of the glycerides are unsaturated fatty acids, 10% by mass or more of the constituent fatty acids of the glycerides are straight-chain saturated fatty acids having 6 to 14 carbon atoms, and the lactones are γ-lactones and/or δ-lactones.

The glycerides contained in the flavor imparting agent may be those contained in the product after oxidation heating of the raw material glycerides, and may optionally contain the additional glycerides above. In the glycerides contained in the flavor-imparting agent, 10% by mass or more of their constituent fatty acids, for example, 30% by mass or more, 60% by mass or more, 80% by mass or more, 90% by mass or more, or all (100% by mass) are straight-chain saturated fatty acids having 6 to 14 carbon atoms. The straight-chain saturated fatty acids having 6 to 14 carbon atoms may be of one type or of two or more types. The straight-chain saturated fatty acids having 6 to 14 carbon atoms include caproic acid, caprylic acid, capric acid, lauric acid, and myristic acid, preferably caprylic acid and/or capric acid, or lauric acid and/or myristic acid. More preferably, caprylic acid and/or capric acid is mentioned, and most preferably, caprylic acid is mentioned.

In cases where unsaturated fatty acids are included as constituent fatty acids in the glycerides, other than straight-chain saturated fatty acids having 6 to 14 carbon atoms, there is a risk that a rancid odor produced by the oxidation of the unsaturated fatty acids may be added to the flavor-imparting agent, so that it is preferable to have a smaller quantity of unsaturated fatty acids. In the flavor-imparting agent of the present invention, it is preferable that 0 to 5% by mass of the constituent fatty acids of the glycerides are unsaturated fatty acids, and it is more preferable that 0 to 3% by mass thereof are unsaturated fatty acids. In the case of including unsaturated fatty acids, it is preferable to have straight-chain unsaturated fatty acids having 16 to 22 carbon atoms, which constitute vegetable oils, and more preferably, one or more selected from oleic acid, linoleic acid, linolenic acid, and erucic acid.

The glycerides contained in the flavor-imparting agent may include glycerides that can be obtained by an esterification reaction of fatty acids and glycerin, an interesterification reaction of fatty acid esters and glycerin, fatty acids and oils and/or fats, or the like, or an interesterification reaction of methyl or ethyl esters of fatty acids and glycerin or oils and/or fats. Also, when unsaturated fatty acids are included, unsaturated bonds may be reduced by hydrogenating fatty acids, methyl or ethyl esters of fatty acids, or glycerides. In addition, hydrogenated coconut oil or palm kernel oil can also be used. Note that it is possible to mix other oils and/or fats, for example, coconut oil, palm kernel oil, palm oil, palm fractionated oil (such as palm olein, palm super olein, palm mid fraction, and palm stearin), shea butter, shea fractionated oil, monkey butter, monkey fractionated oil, illipe butter, soybean oil, rapeseed oil, cottonseed oil, safflower oil, sunflower oil, rice oil, corn oil, sesame oil, olive oil, perilla oil, linseed oil, peanut oil, milk fat, cocoa butter, and other animal and vegetable oils and/or fats, or mixed oils thereof, as well as processed oils and/or fats and the like, within the range where 10% by mass or more of the constituent fatty acids of the glycerides contained in the flavor-imparting agent are straight-chain saturated fatty acids having 6 to 14 carbon atoms.

The glycerides contained in the flavor-imparting agent may include one or more of monoglycerides, diglycerides, or triglycerides, and preferably, 50% by mass or more, 60% by mass or more, 70% by mass or more, 80% by mass or more, 90% by mass or more, 95% by mass or more, or 100% by mass of the glycerides contained in the flavor-imparting agent is triglyceride.

The flavor-imparting agent of the present invention preferably includes a glyceride that has undergone the oxidation heat treatment as described in the aforementioned production method of a flavor-imparting agent. The degree of glyceride oxidation is indicated by the peroxide value and the anisidine value, and these indicators increase due to oxidation.

The glyceride contained in the flavor-imparting agent preferably has a peroxide value of 7.5 or higher. More preferably, the glyceride contained in the flavor-imparting agent has a peroxide value of 10 to 100, 15 to 90, 20 to 80, 25 to 70, or 30 to 60. If the peroxide value of the glyceride is 7.5 or higher, it is possible to confirm that it has undergone a certain level of oxidation. The peroxide value can be measured in accordance with The JOCS Standard Methods for the Analysis of Fats, Oils and Related Materials (edited by the Japan Oil Chemists' Society) 2.5.2.1-2013 "Peroxide Value (Acetic Acid-Isooctane Method)".

In addition, through the oxidation heat treatment, the anisidine value, another oxidation indicator, also increases. The glyceride contained in the flavor-imparting agent may have an anisidine value of 0.8 or more, and it is preferable to have an anisidine value of 1.0 or more. More preferably, the glyceride contained in the flavor-imparting agent may have an anisidine value of 0.9 to 10.0, 1.0 to 8.0, 1.5 to 6.0, 2.0 to 5.5, 2.5 to 5.0, or 3.0 to 4.5. If the anisidine value of the glyceride is 0.8 or higher, it is possible to confirm that it has undergone a certain level of oxidation. The anisidine value can be measured in accordance with The JOCS Standard Methods for the Analysis of Fats, Oils and Related Materials (edited by the Japan Oil Chemists' Society) 2.5.3-2013 "Anisidine Value".

Note that in the same oxidation heat treatment, the peroxide value tends to increase for straight-chain saturated fatty acids having 6 to 10 carbon atoms, while the anisidine value tends to increase for straight-chain saturated fatty acids having 11 to 14 carbon atoms. The glycerides contained in the flavor-imparting agent preferably have a peroxide value of 7.5 or higher and/or an anisidine value of 1.0 or higher. The glycerides that contain straight-chain saturated fatty acids having 6 to 10 carbon atoms as the constituent fatty acids have a peroxide value selected from the range of more preferably 10 to 100, 15 to 90, 20 to 80, 25 to 70, or 30 to 60, and an anisidine value selected from the range of more preferably 1.0 to 8.0, 1.5 to 6.0, 2.0 to 5.5, 2.5 to 5.0, or 3.0 to 4.5. Furthermore, the glycerides that contain straight-chain saturated fatty acids having 11 to 14 carbon atoms have an anisidine value selected from the range of more preferably 1.0 to 8.0, 1.0 to 6.0, 1.0 to 5.0, 1.0 to 4.0, 1.0 to 3.0, or 1.0 to 2.0.

The content of glycerides in the flavor-imparting agent may be 80% by mass or more, preferably 85% by mass or more, 90% by mass or more, 95% by mass or more, or 99% by mass or more, based on the total mass of the flavor-imparting agent. If the content of glycerides is within the aforementioned range, they will be effective as the base material of the flavor-imparting agent.

The lactones having 6 to 14 carbon atoms contained in the flavor-imparting agent are γ-lactones and/or δ-lactones, which may be contained in the oxidation-heated product of the raw material glycerides. The content of the lactones having 6 to 14 carbon atoms in the flavor-imparting agent is 5 ppm or more, preferably 15 ppm or more, 30 ppm or more, 60 ppm or more, 90 ppm or more, 120 ppm or more, 150 ppm or more, 200 ppm or more, 250 ppm or more, or 300 ppm or more, based on the total mass of the flavor-imparting agent. Also, the content of lactones having 6 to 14 carbon atoms in the flavor-imparting agent may be, for example, 600 ppm or less, 500 ppm or less, 400 ppm or less, or 350 ppm or less. If the content of lactones having 6 to 14 carbon atoms is 5 ppm or more, the flavor-imparting agent exhibits a sweet scent and/or milky scent.

There are no particular restrictions on the mass ratio (γ:δ) of γ-lactones and δ-lactones having 6 to 14 carbon atoms contained in the flavor-imparting agent, and for example, it may be 100:0 to 0:100. For example, in the case of the mass ratio (γ:δ) of γ-lactones and δ-lactones having 6 to 10 carbon atoms, it may be 90:10 to 10:90, 80:20 to 20:80, 70:30 to 30:70, 60:40 to 40:60, or 55:45 to 45:55. For example, in the case of the mass ratio (γ:δ) of γ-lactones and δ-lactones having 11 to 14 carbon atoms, it may be 50:50 to 0:100, 40:60 to 0:100, 30:70 to 0:100, 20:80 to 0:100, 10:90 to 0:100, or 5:95 to 0:100.

According to the present invention, a flavor-imparting agent that possesses a sweet scent and/or milky scent is provided. The term "sweet scent" means a scent derived from γ-lactones and/or δ-lactones having 6 to 10 carbon atoms. Moreover, the term "milky scent" means a scent derived from γ-lactones and/or δ-lactones having 11 to 14 carbon atoms.

The flavor-imparting agent may include optional additional ingredients typically added to food, as long as they do not impair the flavor of sweet scent and/or milky scent. The additional ingredients include emulsifiers, antioxidants, alcohol, water, pH adjustors, seasonings, colorants, fragrances, defoamers, sugars, sugar alcohols, stabilizers, and the like. Emulsifiers that can be used include polyglycerin fatty acid esters, polyglycerin condensed ricinoleate, sorbitan fatty acid esters, sorbitol fatty acid esters, sucrose fatty acid esters, propylene glycol fatty acid esters, organic acid monoglycerides, polysorbates, lecithins, and the like. Antioxidants that can be used include tocopherols, tocotrienols, carotenes, polyphenols, ascorbic acid, ascorbic acid derivatives, and the like.

### Examples

### (Raw Material Glycerides)

The following were used as raw material glycerides.
O.D.O ("Medium-Chain Fatty Acid Triglyceride" manufactured by Nisshin OilliO Group, Inc., constituent fatty acids: caprylic acid 75% and capric acid 25%)
O.D.O+Toc (sample of O.D.O added with 500 ppm of soy-derived tocopherol)
C10R ("Medium-Chain Fatty Acid Triglyceride" manufactured by Nisshin OilliO Group, Inc., constituent fatty acids: caprylic acid 30% and capric acid 70%)
Tricaprylin ("Medium-Chain Fatty Acid Triglyceride" manufactured by Nisshin OilliO Group, Inc., constituent fatty acids: caprylic acid about 100%)
Tricaprin ("Medium-Chain Fatty Acid Triglyceride" manufactured by Nisshin OilliO Group, Inc., constituent fatty acids: capric acid about 100%)
Trilaurin ("Trilaurin" manufactured by Tokyo Chemical Industry Co., Ltd., constituent fatty acids: lauric acid about 98%)
Trimyristin ("Trimyristin" manufactured by Tokyo Chemical Industry Co., Ltd., constituent fatty acids: myristic acid about 95%)

### (Oxidation Heat Treatment)

O.D.O at 50.00 g was taken as a sample in a 200 mL beaker, and heated at 180°C using a mantle heater with PID temperature control in the atmosphere. A sweet scent started to emerge once the sample temperature exceeded 140°C. As soon as the sample temperature reached 180°C, at 0, 5, 15, 30, and 60 minutes after, the sample was immediately transferred into a brown bottle that had been nitrogen-replaced, and left covered until it cooled down to 20°C.

For O.D.O+Toc, C10R, tricaprylin, tricaprin, trilaurin, and trimyristin, the treatment was the same as for O.D.O, except that they were transferred into a brown bottle 60 minutes after the sample temperature reached 180°C.

### (Analysis)

For untreated O.D.O., trilaurin, and trimyristin, as well as each sample after treatment, the peroxide value (POV) was measured in accordance with The JOCS Standard Methods for the Analysis of Fats, Oils and Related Materials (edited by the Japan Oil Chemists' Society) 2.5.2.1-2013 "Peroxide Value (Acetic Acid-Isooctane Method)".

For untreated O.D.O., trilaurin, and trimyristin, as well as each sample after treatment, the anisidine value (AnV) was measured in accordance with The JOCS Standard Methods for the Analysis of Fats, Oils and Related Materials (edited by the Japan Oil Chemists' Society) 2.5.3-2013 "Anisidine Value".

For untreated O.D.O., trilaurin, and trimyristin, as well as each sample after treatment, the lactone content was measured by GC/MS under the following conditions.
GC/MS conditions
Column: DB-1HT (15 m×ϕ0.25 mm×0.1 µm)
Temperature ramp condition: 40°C [3 minutes]-4°C/minute-170°C-25°C/minute-370°C [7 minutes]
Carrier gas: Helium
Injector: 370°C, split ratio=20:1
Ion source: EI
Detector: MSD
Solution concentration: 0.1 mg/mL
Injection volume: 1 µL
Analytical method: Quantification (ppm) using C8 to C14 lactone STD solution as an external standard.

The analysis results for peroxide value (POV) and anisidine value (AnV) are shown in Table 1, and the analysis results for lactone content are shown in Table 1 and Fig. 1.

**[Table 1]**

| No. | Raw Material | Heating Time after Reaching 180°C (min) | POV | AnV | γ-C8 (ppm) | γ-C10 (ppm) | γ-C12 (ppm) | δ-C8 (ppm) | δ-C10 (ppm) | δ-C12 (ppm) | δ-C14 (ppm) | Lactone Content (ppm) |
|---|---|---|---|---|---|---|---|---|---|---|---|---|
| 0 | O.D.O | Untreated | 0 | 0 | - | - | - | - | - | - | - | - |
| 1 | O.D.O | 0 | 5.6 | 0.7 | - | - | - | - | - | - | - | - |
| 2 | O.D.O | 5 | 10.5 | 0.9 | 17.8 | - | - | 19.7 | - | - | - | 37.5 |
| 3 | O.D.O | 15 | 23.2 | 1.1 | 27.6 | 18.1 | - | 27.6 | 25.1 | - | - | 98.4 |
| 4 | O.D.O | 30 | 27.3 | 1.7 | 40.6 | 22.0 | - | 38.6 | 29.2 | - | - | 130.4 |
| 5 | O.D.O | 60 | 33.5 | 2.9 | 82.8 | 33.3 | - | 74.5 | 39.7 | - | - | 230.3 |
| 6 | O.D.O+Toc | 60 | 0.3 | 0.9 | - | - | - | - | - | - | - | - |
| 7 | C10R | 60 | 39.4 | 3.2 | 49.5 | 109.5 | - | 49.7 | 113.6 | - | - | 322.3 |
| 8 | Tricaprylin | 60 | 50.2 | 3.6 | 175.8 | - | - | 167.0 | - | - | - | 342.8 |
| 9 | Tricaprin | 60 | 18.5 | 1.6 | - | 65.1 | - | - | 69.2 | - | - | 134.3 |
| 10 | Trilaurin | Untreated | 0 | 0 | - | - | - | - | - | - | - | - |
| 11 | Trilaurin | 60 | 1.4 | 1.1 | - | - | 13.4 | - | - | 51.1 | - | 64.5 |
| 12 | Trimyristin | Untreated | 0 | 0 | - | - | - | - | - | - | - | - |
| 13 | Trimyristin | 60 | 3.1 | 1.0 | - | - | - | - | - | - | 118.3 | 118.3 |

| | | | | | | | | | | | | |
|---|---|---|---|---|---|---|---|---|---|---|---|---|
| * γ-C8: γ-lactone having 8 carbon atoms, γ-C10: γ-lactone having 10 carbon atoms, γ-C12: γ-lactone having 12 carbon atoms δ-C8: δ-lactone having 8 carbon atoms, δ-C10: δ-lactone having 10 carbon atoms, δ-C12: δ-lactone having 12 carbon atoms, δ-C14: δ-lactone having 14 carbon atoms | | | | | | | | | | | | |

In untreated O.D.O, trilaurin, and trimyristin, as well as O.D.O+Toc with added tocopherol as an antioxidant, no lactones were detected. Note that after the oxidation heat treatment, the only lactones that could exist in each sample have 8, 10, 12 and/or 14 carbon atoms, which correspond to the carbon numbers in the constituent fatty acids of the raw material glycerides. Moreover, the quantity of glycerides and the mass ratio of the constituent fatty acids do not substantially change before and after the oxidation heat treatment.

### (Scent Evaluation 1)

The scent of each sample, untreated O.D.O, O.D.O subjected to oxidation heat treatment, O.D.O+Toc, C10R, tricaprylin, and tricaprin (at 20°C), was evaluated based on the following criteria by 10 panelists. The scent of Sample 2 was used as a reference for relative evaluation, rated as 2 points. The results (average scores) are shown in Table 2.
0 points: No sweet scent is felt.
1 point: A faint sweet scent is felt (weaker than Sample 2).
2 points: A sweet scent is felt (equivalent to Sample 2).
3 points: A slightly strong sweet scent is felt (stronger than Sample 2 but about less than twice as much).
4 points: A strong sweet scent is felt (about twice as strong as Sample 2).
5 points: A very strong sweet scent is felt (more than twice as strong as Sample 2).

**[Table 2]**

| No. | Raw Material | Heating Time after Reaching 180°C (min) | Scent | Note |
|---|---|---|---|---|
| 0 | O.D.O | Untreated | 0 | |
| 1 | O.D.O | 0 | 0.2 | |
| 2 | O.D.O | 5 | 2.0 | |
| 3 | O.D.O | 15 | 3.8 | |
| 4 | O.D.O | 30 | 4.4 | |
| 5 | O.D.O | 60 | 5.0 | |
| 6 | O.D.O+Toc | 60 | 0.6 | |
| 7 | C10R | 60 | 4.8 | A faint odor, somewhat reminiscent of paint, was also slightly perceptible. |
| 8 | Tricaprylin | 60 | 4.9 | |
| 9 | Tricaprin | 60 | 3.1 | A faint odor, somewhat reminiscent of paint, was also slightly perceptible. |

Lactones having 8 carbon atoms are produced from fatty acids (caprylic acid) having 8 carbon atoms, while lactones having 10 carbon atoms are produced from fatty acids (capric acid) having 10 carbon atoms. A correlation was observed between the intensity of the sweet scent and the quantity of lactone produced.

### (Scent Evaluation 2)

For untreated samples of trilaurin and trimyristin, and samples of trilaurin and trimyristin subjected to oxidation heat treatment (at 20°C), the presence or absence of scent was evaluated by 10 panelists based on the following criteria. The results (average scores) are shown in Table 3.
0 points: No milk scent is felt.
1 point: A milk scent is felt.

**[Table 3]**

| No. | Raw Material | Heating Time after Reaching 180°C (min) | Scent | Note |
|---|---|---|---|---|
| 10 | Trilaurin | Untreated | 0 | |
| 11 | Trilaurin | 60 | 1 | A scent reminiscent of blue cheese |
| 12 | Trimyristin | Untreated | 0 | |
| 13 | Trimyristin | 60 | 1 | A scent reminiscent of fermented butter |

Lactones having 12 carbon atoms are produced from fatty acids (lauric acid) having 12 carbon atoms, and lactones having 14 carbon atoms are produced from fatty acids (myristic acid) having 14 carbon atoms. Samples containing lactones having 12 or 14 carbon atoms had a milk scent.

### Industrial Applicability

The flavor-imparting agent provided by the present invention can be used to impart flavor to food.

## Claims

1. A method for producing a flavor-imparting agent, comprising:
oxidation heat treatment of glycerides at 140°C or higher to produce lactones having 6 to 14 carbon atoms, thereby preparing a flavor-imparting agent containing 5 mass ppm or more of the lactones having 6 to 14 carbon atoms, wherein
10% by mass or more of constituent fatty acids of the glycerides are straight-chain saturated fatty acids having 6 to 14 carbon atoms, and
the lactones are γ-lactones and/or δ-lactones.

2. The method according to claim 1, wherein 0 to 5% by mass of the constituent fatty acids of the glycerides are unsaturated fatty acids.

3. The method according to claim 1 or 2, wherein 60% by mass or more of the constituent fatty acids of the glycerides are straight-chain saturated fatty acids having 6 to 14 carbon atoms.

4. The method according to any one of claims 1 to 3, wherein the constituent fatty acids of the glycerides are composed only of straight-chain saturated fatty acids having 6 to 14 carbon atoms.

5. The method according to any one of claims 1 to 4, wherein the glycerides are triglycerides.

6. The method according to any one of claims 1 to 5, further comprising: mixing additional glycerides after the oxidation heat treatment, to thereby prepare a flavor-imparting agent containing 5 ppm or more of lactones having 6 to 14 carbon atoms.

7. A flavor-imparting agent comprising:
80% by mass or more of glycerides; and
5 ppm or more of lactones having 6 to 14 carbon atoms, based on a total mass of the flavor-imparting agent, wherein
0 to 5% by mass of constituent fatty acids of the glycerides are unsaturated fatty acids,
10% by mass or more of the constituent fatty acids of the glycerides are straight-chain saturated fatty acids having 6 to 14 carbon atoms, and
the lactones are γ-lactones and/or δ-lactones.

8. The flavor-imparting agent according to claim 7, wherein the glycerides have a peroxide value of 7.5 or higher and/or an anisidine value of 1.0 or higher.

9. The flavor-imparting agent according to claim 7 or 8, wherein 60% by mass or more of the constituent fatty acids of the glycerides are straight-chain saturated fatty acids having 6 to 14 carbon atoms.

10. The flavor-imparting agent according to any one of claims 7 to 9, wherein the constituent fatty acids of the glycerides are composed only of straight-chain saturated fatty acids having 6 to 14 carbon atoms.

11. The flavor-imparting agent according to any one of claims 7 to 10, wherein the glycerides are triglycerides.

12. The flavor-imparting agent according to any one of claims 7 to 11, which has a sweet scent and/or milky scent.

13. A method for imparting flavor to glycerides, comprising:
oxidation heat treatment of glycerides at 140°C or higher to produce 5 mass ppm or more of lactones having 6 to 14 carbon atoms, wherein
10% by mass or more of constituent fatty acids of the glycerides are straight-chain saturated fatty acids having 6 to 14 carbon atoms, and
the lactones are γ-lactones and/or δ-lactones.
